(19) Europäisches Patentamt
European Patent Office
Office européen des brevets

(11) Publication number: **0 067 897**
**A1**

# (12) EUROPEAN PATENT APPLICATION

(21) Application number: 81104766.1

(22) Date of filing: 22.06.81

(51) Int. Cl.³: A 61 K 31/495
A 61 K 9/20
//C07D295/08

(43) Date of publication of application:
29.12.82 Bulletin 82/52

(84) Designated Contracting States:
CH DE FR GB LI NL

(71) Applicant: Tanabe Seiyaku Co., Ltd.
No. 21 Dosho-machi 3-chome Higashi-ku
Osaka-shi Osaka-fu(JP)

(72) Inventor: Nagao, Shosuke
No. 9-9, Midorigaoka 1-chome
Toyonaka-shi Osaka-fu(JP)

(72) Inventor: Noda, Kazuo
No. 24-12, Nakayama-Sakuradai 2-chome
Takarazuka-shi, Hyogo-ken(JP)

(72) Inventor: Yoneyama, Takashi
No. 1770-10, Senriyama-Higashi 4-chome
Suita-shi, Osaka-fu(JP)

(74) Representative: Kraus, Walter, Dr. et al,
Patentanwälte Dres. Kraus & Weisert Irmgardstrasse 15
D-8000 München 71(DE)

**(54) A pharmaceutical composition for use in raised intracranial pressure.**

(57) Tablets adapted for use in reducing increased intracranial pressure are disclosed. Said tablets comprise, as an active ingredient, a propanol derivative of the formula:

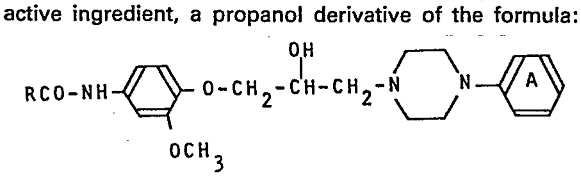

wherein R is alkyl of one to 4 carbon atoms and Ring A is phenyl, halogenophenyl, methylphenyl or trifluoromethylphenyl, or a pharmaceutically acceptable acid addition salt thereof. Said tablets may comprise the active ingredient in combination with an inert diluent, a disintegrator and a lubricant, and if required, may further contain a binder.

EP 0 067 897 A1

Croydon Printing Company Ltd.

TITLE
see front page

This invention relates to a pharmaceutical composition for use in reducing increased intracranial pressure in a warm-blooded animal.

Cerebral edema, which is a well-known complication of various cerebral diseases, induces an increase in intracranial pressure due to compression of neighbouring brain tissues. Moreover, such increased intracranial pressure is known to adversely affect cerebral metabolism, result in disturbances of cerebral circulation and further aggravate cerebral edema. Therefore, the increased intracranial pressure exerts serious adverse effects on patients or is sometimes fatal to them. In this respect, a hypertonic solution (1000 - 1500 milliosmols/kg·$H_2O$) of glycerol or mannitol has been used for therapeutic treatment of patients suffering from increased intracranial pressure. The intravenous infusion of the hypertonic solution raises blood osmolality and creates an osmotic gradient between plasma and brain with resultant net removal of water from brain. Thus, the therapeutic effect of the hypertonic solution on the increased intracranial pressure is based on the hyperosmolar dehydrating effect and, therefore, in order to obtain the desired therapeutic effect, as much as 500 to 1500 ml/man/day of the hypertonic solution must be administered to patients. Such hypertonic solution is also disadvantageous in that its intravenous infusion often causes haematological abnormalities (e.g., disturbance of water-electorolite equilibrium, haemolysis,

decrease in haematocrit level) and is followed by a rebound increase in the intracranial pressure to levels higher than those that existed before therapy.

An object of the present invention is to provide a novel pharmaceutical composition adapted for oral administration to a warm-blooded animal. Another object of the invention is to provide a pharmaceutical composition for use in reducing or treating increased intracranial pressure in a warm-blooded animal, including man, in need of such reduction of intracranial pressure. Other object of the invention is to provide a pharmaceutical composition which enables us to reduce increased intracranial pressure without haematological abnormalities, rebound increase in said pressure and other untoward side effects as seen in the hypertonic solution of glycerol or mannitol. Still other object is to provide a pharmaceutical composition which is useful to treat a warm-blooded animal suffering from a disease or condition attributable to increased intracranial pressure. Further object of the invention will be apparent from the following description and claims.

The pharmaceutical composition of the present invention is concerned with tablets each comprising, as an active ingredient, a propanol derivative of the formula:

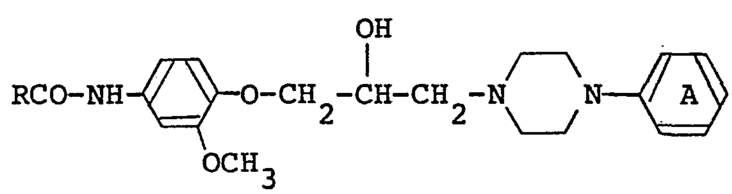

(I)

wherein R is alkyl of one to 4 carbon atoms and Ring A

is phenyl, halogenophenyl, methylphenyl or trifluoromethylphenyl, or a pharmaceutically acceptable acid addition salt thereof. Said active ingredient, i.e., the propanol derivative (I) or its salt, can be used in combination with an inert diluent, a disintegrator and a lubricant, and if required, may be further used together with a binder.

The propanol derivative (I) of the present invention shows a significant decrease in intracranial pressure at a remarkably low dose such as 0.1 - 2 mg/kg of body weight/day. Representative examples of such compound include those of the formula (I) in which R is alkyl having one to 4 carbon stoms (e.g., methyl, ethyl, propyl, butyl), and Ring A is phenyl, methylphenyl (e.g., 2-methylphenyl), chlorophenyl (e.g., 2-chlorophenyl, 3-chlorophenyl, 4-chlorophenyl), fluorophenyl (e.g., 2-fluorophenyl, 3-fluorophenyl, 4-fluorophenyl) or trifluoromethylphenyl (e.g., 3-trifluoromethylphenyl). Among these compounds, preferred group of compounds are those of the formula (I) in which R is methyl and Ring A is phenyl, chlorophenyl or fluorophenyl. More preferred group of compounds are those of the formula (I) in which R is methyl and Ring A is phenyl, 2-chlorophenyl, 2-fluorophenyl or 3-fluorophenyl, and the most preferred compound is 1-(4-acetamido-2-methoxyphenoxy)-3-[4-(3-fluorophenyl)piperazino]-2-propanol. All of these propanol derivatives (I) and a process for preparation thereof have already been disclosed in Japanese Patent Publication (unexamined) No. 21127/1978.

The propanol derivative (I) may be employed either as the free base or in the form of a pharmaceutically acceptable acid addition salt thereof. Pharmaceutically acceptable acid addition salt of the propanol derivative (I) includes, for examples, inorganic acid addition salts such as hydrochloride, phosphate, nitrate and sulfate; and organic acid addition salts such as acetate, lactate, citrate, fumarate, maleate, glycinate, aspartate, methanesulfonate and benzoate. The amount of the propanol derivative (I) or its salt to be contained in each tablets of the invention may vary depending on the age, weight and condition of patients; and the particular diseases to be treated. In general, however, suitable amount of the propanol derivative (I) or its salt to be used is about 5 to about 50 mg, especially about 10 to about 50 mg, per each tablet.

The tablets of the present invention can be made by direct compression, i.e., by the steps of mixing the propanol derivative (I) or its salt with the inert diluent, the disintegrator and the lubricant, and compressing the mixture into a suitable form of tablets. Alternatively, said tablets may be made according to a granulation method, i.e., by the steps of granulating the mixture of the propanol derivative (I) or its salt, the disintegrator and the inert diluent with the aid of the binder, adding the lubricant thereto and then compressing the mixture into tablets.

Various disintegrators and lubricants can be used in

making the tablets of the invention. For example, carboxymethylcelllulose, calcium carboxymethylcellulose, alginic acid or its salt are preferably used as the disintegrator, and suitable examples of the lubricant include stearic acid, magnesium stearate, calcium stearate and talc. On the other hand, the inert diluent which can be used in the invention includes, for example, calcium citrate, calcium phosphate, sucrose, dextrose, lactose, mannitol and sorbitol. Starch and crystalline cellulose may be used either as the disintegrator or the inert diluent because they have both functions. The binder which may be optionally used to impart a pertinent cohesiveness to the tablet includes, for example, natural polymers such as gelatin, dextrin or acacia, synthetic polymers such as polyvinylpyrrolidone and cellulose derivatives such as hydroxypropylcellulose, methylcellulose or sodium caboxymethylcellulose. Compression of these ingredients into tablets, granulation as well as compression of the granules of said ingredients can be readily accomplished by the use of conventional tablet machines, mixing machines for granulation, kneader, granulator, dryer, etc. Moreover, each tablets of the present invention may be formed into various shapes and sizes. For example, they may be either discoid, round, oval or oblong in shape. Each tablets should weigh about 50 to about 500 mg, especially about 70 to about 500 mg, and have the hardness of about 4 to about 20 kg when measured by Schleuniger's hardness tester Model 2E.

The tablets of the present invention for use in the therapeutic treatment of increased intracranial pressure should release the active ingredient, i.e., the propanol derivative (I) or its salt, from the tablet matrix as efficiently as possible to allow for its rapid dissolution after oral administration thereof by patients and should preferably disintegrate within about 10 minutes, especially within about 2 minutes, when tested by tablet disintegration apparatus. For this purpose, it is suitable to use about one to about 40 w/w % of the disintegrator, about 0.3 to about 2 w/w % of the lubricant and 0 to about 10 w/w % of the the binder. Particularly, in making the tablets according to the direct compression method, it is preferred to use about one to about 30 w/w %, more preferably about 5 to about 15 w/w %, of the disintegrator. On the other hand, when the tablets are made according to the wet granulation method, it is preferred to use about 3 to about 40 w/w %, more preferably about 5 to about 25 w/w %, of the disintegrator. The use of the binder is not critical in the invention if the tablets are made according to the direct compression method. In addition, even in making the tablets according to the wet granulation method, the use of too much binder will make a hard tablet which will not disintegrate easily. Preferred amount of the binder to be used from this point of view is about one to about 4 w/w %. The inert diluent is used in an amount sufficient to bring the tablet up to 100 w/w %,

i.e., to increase the bulk in order to make the tablet a practical size for compression. Such amount of the diluent may be readily decided on the weight of each tablet to be made as well as on the amount of each one of the active ingredient, disintegrator, lubricant and binder used.

The pharmaceutical composition (i.e., tablets) of the present invention can be used for therapeutic treatment or prognosis of a warm-blooded animal, including man, suffering from increased intracranial pressure due to various cerebral diseases such as cerebral infarction, cerebral thrombosis, cerebral embolism, cerebral hemorrhage, subarachnoid hemorrhage, head injury, cerebral tumor, cerebral edema, encephalomyelitis and the like. The propanol derivative (I) may be also used to ameliorate such various cerebral diseases as mentioned above. Further, since the propanol derivative (I) has a potent cerebral blood-flow-increasing activity in addition to its effect on the intracranial pressure, it can also be used to improve the cerebral circulation, especially in ischemic areas in brain.

The propanol derivative (I) shows significant decrease in intracranial pressure even when administered at such a remarkably low dose as 0.5 mg/kg of body weight; said therapeutic effect lasts for a long period of time such as for more than 5 - 6 hours; and its use is not associated with rebound increase in intracranial pressure. Unlike those of the hypertonic solutions of glycerol or mannitol, the therapeutic

effect of the propanol derivative (I) of the present invention is not ascribable to the cerebral dehydrating effect. The propanol derivative (I) may be used for the treatment or prophylaxis of increased intracranial pressure during and after brain surgical operation. Moreover, the propanol derivative (I) may be used for improving impaired consciousness, nervous disturbance and subjective symptoms (e.g., headache, nausea, vomiting) which are attributed to the cerebral diseases such as those mentioned hereinbefore. Further, whereas it is known that cerebral edema induces an increase in intracranial pressure and increased intracranial pressure serves to cause a build-up of edema which in turn impairs cerebral circulation, the propanol derivative (I) can interrupt such vicious cycle of cerebral diseases because of its potent intracranial pressure-lowering effect and ameliorate the cerebral circulatory impairment. The toxicity of the propanol derivative (I) of the invention is low. For example, when the acute toxicity of a test compound is estimated a week after oral administration thereof to male mice, $LD_{50}$ of 1-(4-acetamido-2-methoxyphenoxy)-3-[4-(3-fluorophenyl)piperazino]-2-propanol of the invention is about 1500 mg/kg.

Concomitantly, the propanol derivative (I) of the present invention is prepared in accordance with the method described in Japanese Patent Publication (unexamined) No. 21127/1978, i.e., by reacting a 3-(4-acylamino-2-methoxyphenoxy)-1,2-epoxypropane with a piperazine derivative of

the formula:

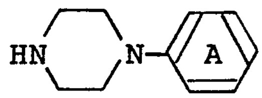

wherein Ring A is the same as defined above.

Example 1

25 g of 1-(4-acetamido-2-methoxyphenoxy)-3-[4-(3-fluorophenyl)piperazino]-2-propanol hydrochloride, 135.2 g of calcium citrate, 18 g of carboxymethylcellulose and 1.8 g of magnesium stearate were mixed thoroughly. Then, said mixture was compressed into tablets (average weight: 180 mg per each tablets), using a 8 mm diameter concave punch. The tablets thus obtained had the hardness of 7.5 kg when measured by Schleuniger's hardness tester.

The disintegration test was carried out according to the method prescribed in The Pharmacopoeia of Japan 9th-Edition page 706 (English version). As a result, the tablets obtained above disintegrated within one minute.

Example 2

25 g of 1-(4-acetamido-2-methoxyphenoxy)-3-[4-(3-fluorophenyl)piperazino]-2-propanol hydrochloride, 135.2 g of calcium citrate, 18 g of corn starch and 1.8 g of magnesium stearate were mixed thoroughly. Then, said mixture was compressed into tablets (average weight: 180 mg per each tablets), using a 8 mm diameter concave punch. When measured

in the same manner as described in Example 1, the tablets thus obtained had the hardness of 7.3 kg and disintegrated within one minute.

Example 3

25 g of 1-(4-acetamido-2-methoxyphenoxy)-3-[4-(3-fluorophenyl)piperazino]-2-propanol hydrochloride, 139 g of lactose and a solution of 5 g of polyvinylpyrrolidone in ethanol were mixed thoroughly, and the mixture was passed through a 16 mesh screen. The granules obtained were dried, and mixed well with 9 g of calcium carboxymethylcellulose and 2 g of magnesium stearate. Said mixture was then compressed into tablets (average weight: 180 mg per each tablets), using 8 mm diameter concave punch. When measured in the same manner as described in Example 1, the tablets thus obtained had the hardness of 7.0 kg and disintegrated within 2 minutes.

Example 4

25 g of 1-(4-acetamido-2-methoxyphenoxy)-3-[4-(3-fluorophenyl)piperazino]-2-propanol hydrochloride, 139 g of lactose and a solution of 5 g of polyvinylpyrrolidone in ethanol were mixed thoroughly, and the mixture was passed through a 16 mesh screen. The granules obtained were dried, and mixed well with 9 g of corn starch and 2 g of magnesium stearate. Said mixture was then compressed into tablets (average weight: 180 mg per each tablets), using a 8 mm diameter concave punch. When measured in the same manner

as described in Example 1, the tablets thus obtained had the hardness of 7.0 kg and disintegrated within 3 minutes.

Example 5

25 g of 1-(4-acetamido-2-methoxyphenoxy)-3-[4-(3-fluorophenyl)piperazino]-2-propanol hydrochloride, 141 g of lactose and a solution of 3 g of hydroxypropylcellulose in water were mixed thoroughly, and the mixture was passed through a 16 mesh screen. The granules obtained were dried, and mixed well with 9 g of calcium carboxymethylcellulose and 2 g of magnesium. Said mixture was then compressed into tablets (average weight: 180 mg per each tablets), using a 8 mm diameter concave punch. When measured in the same manner as described in Example 1, the tablets thus obtained had the hardness of 6.3 kg and disintegrated within 2 minutes.

Example 6

Tablets (average weight: 180 mg per each tablets) of 8 mm in diameter were made in the same manner as described in Example 1 except that 67 g of crystalline cellulose, 68.2 g of calcium phosphate, 18 g of alginic acid and 1.8 g of stearic acid were used instead of calcium citrate, carboxymethylcellulose and magnesium stearate.

Example 7

Tablets (average weight: 180 mg per each tablets) of 8 mm in diameter were made in the same manner as described in Example 1 except that 67 g of sucrose mixture, 66.4 g of

dextrose mixture, 18 g of sodium alginate and 3.6 g of talc were used instead of calcium citrate, carboxymethylcellulose and magnesium stearate.

Example 8

Tablets (average weight: 180 mg per each tablets) of 8 mm in diameter were made in the same manner as described in Example 1 except that 67 g of mannitol, 66.4 g of sorbitol and 3.6 g of stearic acid were used instead of calcium citrate and magnesium stearate.

Example 9

Tablets were made in the same manner as described in Example 5 except that 5 g of gelatin were used instead of hydroxypropylcellulose.

Example 10

Tablets were made in the same manner as described in Example 5 except that 3 g of dextrin were used instead of hydroxypropylcellulose.

Example 11

Tablets were made in the same manner as described in Example 5 except that 3 g of acacia were used instead of hydroxypropylcellulose.

Example 12

Tablets were made in the same manner as described in Example 5 except that 3 g of methylcellulose were used instead of hydroxypropylcellulose.

Example 13

Tablets were made in the same manner as described in Example 5 except that 3 g of sodium carboxymethylcellulose were used instead of hydroxypropylcellulose.

Experiment

Male SD-strain rats weighing 500 to 800 g (each group consisiting of 5 rats) were fasted for about 20 hours, and anesthetized with urethane. 1-(4-acetamido-2-methoxyphenoxy)-3-[4-(3-fluorophenyl)piperazino]-2-propanol (free base) was administered orally to the rats at a dose of 8 mg/kg. Intracranial pressure (i.e., cerebrospinal fluid pressure in the cisterna magna) was continuously monitored via a cannula inserted in the cisterna magna. The cannula was connected to a transducer, and recordings were made on graph paper. The effect of said test compound on the intracranial pressure was estimated in terms of the increase or decrease in intracranial pressure, which was calculated in accordance with the following formula:

$$\text{Changes (\%) in intracranial pressure} = \left[ \frac{\text{Intracranial pressure (mm } H_2O\text{) measured after administration of test compound}}{\text{Intracranial pressure (mm } H_2O\text{) measured before administration of test compound}} \right] \times 100$$

The results are shown in the following Table.

Table

| Changes in intracranial pressure (%) | | | | | | |
|---|---|---|---|---|---|---|
| Time after adminstration of test compound (minutes) | | | | | | |
| 0* | 20 | 60 | 120 | 180 | 240 | 300 |
| 100 | 85.4 | 83.5 | 77.7 | 76.5 | 75.6 | 77.9 |

Note: * : the intracranial pressure measured before administration of test compound was 70 ± 3.1 mm $H_2O$ (0 minute).

Preparation of the propanol derivative (I)

(1) 1.0 g of 3-(4-acetamido-2-methoxyphenoxy)-1,2-epoxypropane is dissolved in 40 ml of ethanol, and 750 mg of 4-phenylpiperazine are added thereto. The mixture is stirred at room temperature for 18 hours. Then, the mixture is concentrated under reduced pressure to remove ethanol. The residue is recrystallized from a mixture of ethyl acetate and methanol, whereby 1.55 g of 1-(4-acetamido-2-methoxyphenoxy)-3-(4-phenylpiperazino)-2-propanol are obtained as colorless needles. M.p. 154° - 156°C

Hydrochloride: M.p. 242° - 244°C (decomp.)

Methanesulfonate: M.p. 191° - 193°C

(2) 800 mg of 3-(4-acetamido-2-methoxyphenoxy)-1,2-epoxypropane are dissolved in 40 ml of ethanol, and 670 mg of 4-(2-fluorophenyl)piperazine are added thereto. The mixture

is refluxed for 4 hours. Then, the mixture is concentrated under reduced pressure to remove ethanol. The residue is recrystallized from a mixture of isopropyl alcohol and isopropyl ether, whereby 770 mg of 1-(4-acetamido-2-methoxyphenoxy)-3-[4-(2-fluorophenyl)piperazino]-2-propanol are obtained as colorless granules.

The mother liquor obtained after recrystallization is concentrated to dryness. The residue is purified by silica gel chromatography (Solvent: 2 % ethanol-chloroform), and then recrystallized from a mixture of isopropyl alcohol and isopropyl ether. 460 mg of 1-(4-acetamido-2-methoxyphenoxy)-3-[4-(2-fluorophenyl)piperazino]-2-propanol are further obtained. M.p. 143.5° - 144.5°C

The following compounds are obtained in the same manner as described in paragraph (1) or (2)

1-(4-acetamido-2-methoxyphenoxy)-3-[4-(2-methylphenyl)-piperazino]-2-propanol, M.p. 126.5° - 128°C

1-(4-acetamido-2-methoxyphenoxy)-3-[4-(4-chlorophenyl)-piperazino]-2-propanol, M.p. 177° - 178.5°C

1-(4-acetamido-2-methoxyphenoxy)-3-[4-(3-chlorophenyl)-piperazino]-2-propanol, M.p. 141° - 143.5°C

1-(4-acetamido-2-methoxyphenoxy)-3-[4-(2-chlorophenyl)-piperazino]-2-propanol, M.p. 137.5° - 140°C

1-(4-acetamido-2-methoxyphenoxy)-3-[4-(4-fluorophenyl)-piperazino]-2-propanol, M.p. 175.5° - 176.5°C

1-(4-acetamido-2-methoxyphenoxy)-3-[4-(3-fluorophenyl)-piperazino]-2-propanol, M.p. 144.5° - 146°C

Hydrochloride: M.p. 224° - 226°C

1-(4-acetamido-2-methoxyphenoxy)-3-[4-(3-trifluoromethylphenyl)piperazino]-2-propanol, M.p. 125° - 127°C

WHAT WE CLAIM IS:

1. A tablet for use in reducing increased intracranial pressure which comprises, as an active ingredient, a propanol derivative of the formula:

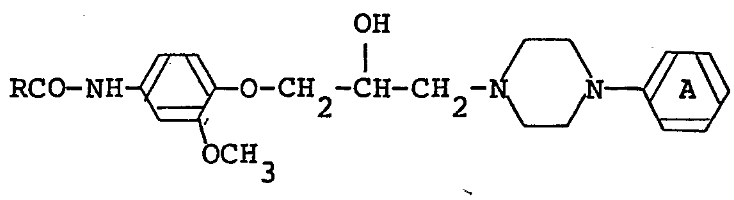

(I)

wherein R is alkyl of one to 4 carbon atoms and Ring A is phenyl, halogenophenyl, methylphenyl or trifluoromethylphenyl, or a pharmaceutically acceptable acid addition salt thereof.

2. The tablet of Claim 1 which has a hardness of 4 to 20 kg and weighs 50 to 500 mg.

3. The tablet of Claim 1 or 2 which contains 5 to 50 mg of the propanol derivative (I) or a pharmaceutically acceptable acid addition salt thereof.

4. The tablet of Claim 1 or 2 which consists essentially of 5 to 50 mg of the propanol derivative (I) or a pharmaceutically acceptable acid addition salt thereof, one to 40 w/w % of a disintegrator, 0.3 to 2 w/w % of a lubricant, 0 to 10 w/w % of a binder and a sufficient amount of an inert diluent to bring said tablet up to 100 w/w %.

5. The tablet of Claim 1 or 2 which consisits essentially of 5 to 50 mg of the propanol derivative (I) or a pharmaceutically acceptable acid addition salt thereof, one to 30 w/w % of a disintegrator, 0.3 to 2 w/w % of a

lubricant and a sufficient amount of an inert diluent to bring said tablet up to 100 w/w %.

6. The tablet of Claim 5, wherein the disintegrator is used in an amount of 5 to 15 w/w %.

7. The composition of Claim 1 or 2 which consists essentially of 5 to 50 mg of the propanol derivative (I) or a pharmaceutically acceptable acid addition salt thereof, 3 to 40 w/w % of a disintegrator, 0.3 to 2 w/w % of a lubricant, one to 4 w/w % of a binder and a sufficient amount of an inert diluent to bring said tablet up to 100 w/w.

8. The tablet of Claim 7, wherein the disintegrator is used in an amount of 5 to 25 w/w %.

9. The tablet of Claim 4,5,6,7 or 8, wherein the propanol derivative (I) or a pharmaceutically acceptable acid addition salt thereof is used in an amount of 10 to 50 mg per tablet.

10. The tablet of Claim 1,2,3,5 or 6, wherein at least one member selected from the group consisting of crystalline cellulose, calcium citrate, calcium phosphate, starch, sucrose, dextrose, lactose, mannitol and sorbitol is used as the diluent, at least one member selected from the group consisting of carboxymethylellulose, calcium carboxymethylcellulose, starch, crystalline cellulose and alginic acid or its salt is used as the disintegrator, and at least one member selected from the group consisting of stearic

acid, magnesium stearate, calcium stearate and talc is used as the lubricant.

11. The tablet of Claim 4, 7 or 8, wherein at least one member selected from the group consisting of crystalline cellulose, calcium citrate, calcium phosphate, starch, sucrose, dextrose, lactose, mannitol and sorbitol is used as the diluent, at least one member selected from the group consisting of carboxymethylcellulose, calcium carboxymethylcellulose, starch, crystalline cellulose and alginic acid or its salt is used as the disintegrator, at least one member selected from the group consisting of stearic acid, magnesium stearate, calcium stearate and talc is used as the lubricant, and at least one member selected from the group consisting of gelatin, dextrin, acacia, polyvinylpyrrolidone, hydroxypropylcellulose, methylcellulose and sodium carboxymethylcellulose is used as the binder.

12. The tablet of Claim 1,2,3,4,5,6,7,8,9,10 or 11, wherein the propanol derivative (I) is selected from the group consisting of 1-(4-acetamido-2-methoxyphenoxy)-3-(4-phenylpiperazino)-2-propanol, 1-(4-acetamido-2-methoxyphenoxy-3-[4-(chlorophenyl)piperazino]-2-propanol and 1-(4-acetamido-2-methoxyphenoxy)-3-[4-(fluorophenyl)piperazino]-2-propanol.

13. The tablet of 1,2,3,4,5,6,7,8,9,10 or 11, wherein the propanol derivative (I) is selected from the group consisting of 1-(4-acetamido-2-methoxyphenoxy)-3-(4-phenylpiperazino)-2-propanol, 1-(4-acetamido-2-methyoxyphenoxy)-3-[4-(2-chlorophenyl)piperadino]-2-propanol, 1-(4-acetamido-2-methoxyphenoxy)-3-[4-(2-fluorophenyl)piperazino]-2-propanol and 1-(4-acetamido-2-methoxyphenoxy)-3-[4-(3-fluorophenyl)-piperazino]-2-propanol.

14. The tablet of Claim 1,2,3,4,5,6,7,8,9,10 or 11, wherein the propanol derivative (I) is 1-(4-acetamido-2-methoxyphenoxy)-3-[4-(3-fluorophenyl)piperazino]-2-propanol.

15 A process for preparing tablets adapted for use in reducing increased intracranial pressure, which comprises mixing a propanol derivative of the formula:

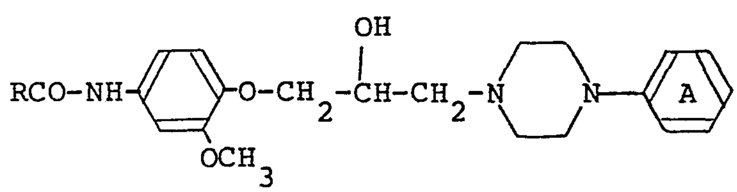

(I)

wherein R is alkyl of one to 4 carbon atoms and Ring A is phenyl, halogenophenyl, methylphenyl or trifluoromethylphenyl, or a pharmaceutically acceptable acid addition salt thereof with an inert diluent, a disintegrator and a lubricant, and compressing the mixture into tablets.

16 A process for preparing tablets adapted for use in reducing increased intracranial pressure, which comprises mixing a propanol derivative of the formula:

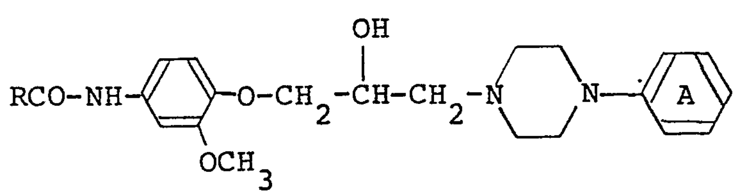

(I)

wherein R is alkyl of one to 4 carbon atoms and Ring A is phenyl, halogenophenyl, methylphenyl or trifluoromethylphenyl,

or a pharmaceutically acceptable acid addition salt thereof with an inert diluent and a disintegrator, granulating the mixture with the aid of a binder, adding a lubricant thereto and then compressing the mixture into tablets.

17 The process according to Claim 15, wherein the mixture of the propanol derivative (I) or its salt, the inert diluent, the disintegrator and the lubricant is compressed into tablets each weighing 50 to 500 mg and having the hardness of 4 to 20 kg.

18 The process according to Claim 16, wherein the mixture of the lubricant and the granules containing the propanol derivative (I) or its salt, the inert diluent, the disintegrator and the binder are compressed into tablets each weighing 50 to 500 mg and having the hardness of 4 to 20 kg.

19 The process according to Claim 15 or 17, wherein the propanol derivative (I) or its salt is used in an amount sufficient to provide 5 to 50 mg per each tablet.

20 The process according to Claim 16 or 18, wherein the propanol derivative (I) or its salt is used in an amount sufficient to provide 5 to 50 mg per each tablet.

21 The process according to Claim 15, 17 or 19, wherein at least one member selected from the group consisting of crystalline cellulose, calcium citrate, calcium phosphate, starch, sucrose, dextrose, lactose, mannitol and sorbitol is used as the diluent, at least one member selected from the

group consisting of caboxymethylcellulose, calcium carboxymethylcellulose, starch, crystalline cellulose and alginic acid or its salt is used as the disintegrator, and at least one member selected from the group consisting of stearic acid, magnesium stearate, calcium stearate and talc is used as the lubricant.

22. The process according to Claim 16, 18 or 20, wherein at least one member selected from the group consisting of crystalline cellulose, calcium citrate, calcium phosphate, starch, sucrose, dextrose, lactose, mannitol and sorbitol is used as the diluent, at least one member selected from the group consisting of carboxymethylcellulose, calcium carboxymethylcellulose, starch, crystalline cellulose and alginic acid or its salt is used as the disintegrator, at least one member selected from the group consisting of stearic acid, magnesium stearate, calcium stearate and talc is used as the lubricant, and at least one member selected from the group consisting of gelatin, dextrin, acacia, polyvinylpyrrolidone, hydroxypropylcellulose, methylcellulose and sodium carboxymethylcellulose is used as the binder.

0067897

European Patent Office

# EUROPEAN SEARCH REPORT

Application number

EP 81104766.1

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| X | PATENT ABSTRACTS OF JAPAN, unexamined applications, Section C, Vol. 2, No. 67, May 20, 1978,<br>The Patent Office Japanese Government, page 544 C 78<br>+ Kokai No. 53-21127 (TANABE) +<br>-- | 1 | A 61 K 31/495<br>A 61 K 9/20//<br>C 07 D 295/08 |
| | US - A - 4 000 274 (RETH, MENTRUP, SCHROMM, DANNEBERG)<br>+ Abstract; columns 1,2,18,19 +<br>-- | 1-22 | |
| | US - A - 3 951 983 (DANILEWICZ, KEMP, SNAREY, WRIGHT)<br>+ Columns 1,2 +<br>---- | 1 | TECHNICAL FIELDS SEARCHED (Int. Cl.3)<br>A 61 K 31/00<br>C 07 D 295/00 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 08-01-1982 | MAZZUCCO |

EPO Form 1503.1 06.78